# EUROPÄISCHE PATENTANMELDUNG

(11) **EP 0 941 721 A1**
(43) Veröffentlichungstag der Anmeldung: **15.09.1999**
(21) Anmeldenummer: 98104320.1
(22) Anmeldetag: 10.03.1998
(51) Int. Cl.: A61F 5/00

(54) **Geradehalter und Osteoporose-Orthese**

(71) Anmelder: VIBROSTATIC MARKETING & FINANCE CORPORATION LIMITED, 9490 Vaduz (LI)
(72) Erfinder: Benckhuijsen, Gerrit Jan, 9492 Eschen (LI)
(74) Vertreter: Büchel, Kurt F., Dr.

(57) **Zusammenfassung**

Geradehalter, insbesondere für Patienten mit Osteoporose-Beschwerden, besteht aus zwei über die Vorderseite der Schultern eines Patienten verlaufenden Schulterbändern (1,1'), deren obere, im Nackenbereich (7) des Patienten liegende Enden (2,2') mit dem oberen Ende eines in Längsrichtungüber den Rücken verlaufenden Rückenbandes (8) verbunden sind, und die unter den Achseln des Patienten auf seinen Rücken verlaufen, während die beiden unteren Enden (3,3') der Schulterbänder (1,1') an einer zumindest den Lendenbereich des Patienten umgreifenden Bandage (6) befestigt sind. Die Befestigung entweder der oberen (2,2') oder der unteren (3,3') Enden ist vorzugsweise lösbar ausgebildet, insbesondere mittels eines Klettverschlusses. Die Bandage (6) ist vorzugsweise als - gegebenenfalls mit Verstärkungen (4,5) versehbare - Rumpf-Orthese ausgebildet. Geradehalter und Bandage (6) weisen zweckmässigerweise wenigstens auf der dem Körper des Patienten zugewandten Innenseite ein Textilmaterial auf, das durch die Ausbildung von elektrostatischen Ladungen den Sauerstoff-Partialdruck der darunter liegenden Muskulatur erhöht.

## Beschreibung

Die Erfindung betrifft ein therapeutisches Hilfsmittel zur aktiven Stützung und Geradehaltung der Rückenwirbelsäule vor allem im Brust- und Lendenbereich. Aufgrund seiner speziellen Konstruktion ermöglicht dieses Hilfsmittel eine flexible, die Muskulatur stimulierende und schmerzlindernde Stützung des Skeletts. Seine Anwendung ist daher speziell zur Behandlung von Patienten mit Schäden des Stützskeletts, insbesondere bei akuten oder chronischen Wirbelsäulenschäden, beispielsweise bei Osteoporose-Patienten angezeigt.

Die in der einschlägigen Literatur beschriebenen, in der Orthopädie gebräuchlichen Rumpforthesen werden durchwegs auch für die Behandlung von Osteoporosepatienten empfohlen. Die bekannten Modelle reichen vom starren, passiven Geradehalter, der bei älteren Patienten nichts bewirken kann, über wirkungslose, zu schwache Leibbinden, bis hin zu regelrechten "Stahlpanzern". Letztere werden, ebenso wie Orthesen mit starren Pelotten von den Patienten häufig nicht getragen.

Nun ist aber gerade bei der durch die Osteoporose hervorgerufenen Skeletterkrankung eine kräftige Muskulatur von besonderer Bedeutung. Eine kräftige Muskulatur stützt das Skelett gut und erlaubt dem Patienten weitgehende Bewegungsmöglichkeiten, die wiederum zur Stärkung der Muskulatur beitragen. Starre Korsette verstärken die Inaktivität der Patienten und damit die Osteoporose.

Die Erfindung hat sich daher die Aufgabe gestellt, einen von Patienten sehr gut akzeptierten Geradehalter für den Oberkörper von an akuten oder chronischen Defekten des Stützskeletts leidenden, vor allem an Osteoporose erkrankten, Patienten zu schaffen, der neben der Haltefunktion auch und gerade eine Kräftigung der Muskulatur bewirkt, um so die körpereigenen Hilfskräfte ins Spiel zu bringen, und dies vorzugsweise nicht nur im Bereich der Hals- und Brustwirbelsäule, sondern auch und gerade im Bereich der Lendenwirbelsäule.

Diese Aufgabe wird erstmals überraschend durch die Kombination der im Kennzeichen des Anspruches 1 wiedergegebenen Merkmale gelöst, vorzugsweise in weiterer Kombination mit dem Kennzeichen des Anspruches 7. Vorteilhafte bzw. alternative Weiterbildungen der Erfindung sind in den abhängigen Ansprüchen unter Schutz gestellt. Die therapeutisch ungünstige passive Wirkung bisheriger Bandagen wird damit durch eine elastische, aktive Korrekturmöglichkeit und Stützfunktion ersetzt.

In der DE-PS-2143781 ist bereits eine den Bereich der Nieren, sowie der Bandscheiben, insbesondere im Lendenbereich, umgreifende und stützende Rumpf-Orthese beschrieben, die aber für die Behandlung der Osteoporose nicht ausreicht. Der erfindungsgemässe Geradehalter hingegen, insbesondere in Verbindung mit einer den Lendenbereich umgreifenden Bandage, vorzugsweise mit der genannten Rumpf-Orthese, ergibt - auch gegenüber an sich bekannten Geradehaltern - einen erstaunlich synergistischen Effekt hinsichtlich Schmerzlinderung und Wohlbefinden der Patienten einerseits und teilweisem Heilerfolg andererseits, welcher Effekt in klinischen Untersuchungen durch Prof. Dr. Jürgen Franke an der Klinik für Orthopädie, Sportmedizin und Osteologie der Heinrich-Mann-Klinik in Bad Liebenstein (DE) nachgewiesen werden konnte.

Die bevorzugte Ausführungsform der vorliegenden Erfindung ist daher eine Osteoporose-Orthese, die aus zwei Teilen besteht, nämlich einerseits aus der erwähnten Rumpf-Orthese und andererseits aus dem Geradehalter. Die Rumpforthese weist, wie erwähnt, neben ihrer idealen Stützfunktion auch die in der EP-A1-585.253 beschriebene Wirkung auf, den Sauerstoffpartialdruck im darunter liegenden Muskelgewebe zu erhöhen, wenn sie - wie dies für viele Anwendungen bevorzugt ist - zumindest auf der dem Körper des Patienten zugewandten Seite ein Textilmaterial aufweist, das zu mindestens 50% aus wenigstens einer der folgenden Fasern besteht: Polyvinylchlorid-, Polyester- und Acryl-Fasern, Polypropylen-, Polyamid- oder Wolle-Fasern mit einem Anteil von weniger als 10% Baumwoll-, Zellwoll- und/oder Seiden-Fasern, insbesondere aus einer Zusammensetzung von wenigstens 50% Polyvinylchlorid-Fasern, vorzugsweise aus mehr als 75% Polyvinylchlorid- und maximal 25% Acryl- und/oder Polyester-Fasern.

Der beim Tragen durch den Patienten mit der Rumpforthese verbundene, grundsätzlich aber von ihr abnehmbar ausgebildete Geradehalter ist dorsal in wenigstens drei Stufen, bevorzugt aber stufenlos verstellbar ausgebildet, um leichter einerseits verschiedenen Körpergrössen, andererseits zunehmenden Heilungserfolgen angepasst werden zu können. Auch die Bänder dieses Geradehalters sind so ausgebildet, dass sie die in der EP-A1-585.253 beschriebene Wirkung aufweisen, d.h. sie enthalten wenigstens auf der dem Körper zugewandten Seite ein Textilmaterial, welches vorzugsweise dem zuvor für die Rumpforthese beschriebenen entspricht. Dies bewirkt eine wertvolle Verbesserung der Durchblutung des darunter liegenden Gewebes, insbesondere der bei Osteoporose zumeist atrophischen, verspannten Muskulatur. Der erfindungsgemässe Geradehalter sollte daher direkt auf der Haut getragen werden, um die gewünschte Erhöhung des Sauerstoffpartialdrucks in der Muskulatur im Bereich der Hals- und Brustwirbelsäule zu erzielen. Nur mit einer ausreichenden Sauerstoffversorgung können die Muskelzellen optimal arbeiten. Und nur eine optimal versorgte Muskulatur kann ihrer Funktion gerecht werden, also das Skelett gut stützen.

Die zweiteilige Orthese verbessert ausserdem die Statik des Brustkorbs durch Wiederherstellung der Ballonfunktion des Abdomens (Entlastung der schmerzhaften Lendenwirbelsäule) und erlaubt dadurch auch nach Wirbelbrüchen eine frühzeitige Mobilisation des Patienten. Sie wird daher in schweren Fällen und in der akuten Phase ständig getragen; in der chronischen Phase genügt ein zeitweiliges Anlegen, insbesondere während allfällig unvermeidlicher Belastungen. Die Konzeption der erfindungsgemässen zweiteiligen Orthese ergibt eine hohe Akzeptanz, auch durch ältere Patienten, weil sie schnell angelegt werden kann, einen guten Tragekomfort bietet (im Gegensatz zu marktüblichen Pelotten, die oft schmerzhafte Druckstellen an den Knochen hervorrufen), die Atmung nicht beeinträchtigt, hautfreundlich ist und keine schädlichen Nebenwirkungen aufweist. Ausserdem bringt sie in so gut wie allen Fällen eine rasche Schmerzlinderung, insbesondere durch die schnellere Mobilisation des Bewegungsapparates nach frischen Wirbelkompressionen, sowie in der chronischen Osteoporose-Phase durch Verbesserung der Statik über die Bauchblase, und durch die Funktion des Geradehalters bei schweren Kyphosen infolge zahlreicher Wirbelfrakturen ("Witwenbuckel") und Reiben der Rippen auf dem Beckenkamm. Nach frischen, stark schmerzhaften Wirbelfrakturen kann die Bettruhe von 2-3 Wochen auf 1 Woche reduziert werden, wodurch der Inaktivität vorgebeugt wird. Schliesslich soll auch nicht unerwähnt bleiben, dass die Schmerzlinderung nachweislich zu einer drastischen Reduktion des Medikamenten-Verbrauchs führt.

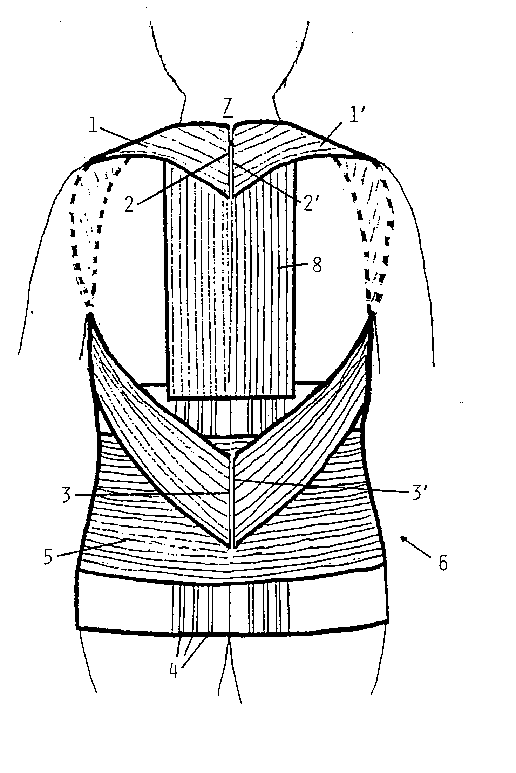

Die Erfindung wird anhand der Zeichnung beispielhaft näher erläutert. Die Figur zeigt die Ansicht des Rückens eines Patienten mit angelegter Osteoporose-Orthese. Zwei Schulterbänder 1,1' verlaufen - beginnend mit ihren oberen Enden 2,2' am Nackenbereich 7 des Patienten über dessen Schultern und Schlüsselbeine auf seine Brustseite nach vorne, und unter seinen Achseln hindurch wieder auf seinen Rücken. Ihre unteren Enden 3,3' sind an der - vorzugsweise dem Körper abgewandten, äusseren - Seite einer den Lendenbereich des Patienten umgreifenden Bandage 6 befestigt.

Die oberen Enden 2,2' der Schulterbänder 1,1' sind ihrerseits am oberen Ende eines senkrecht über den Rücken verlaufenden Rückenbandes 8 befestigt. Wenigstens eine der beiden genannten Befestigungen der Schulterbänder 1,1' (an der Bandage 6 und/oder am Rückenband 8) ist vorzugsweise lösbar ausgebildet, insbesondere mittels (nicht dargestelltem) Klettverschluss. Die andere Befestigung kann, z.B. durch Nähen, unlösbar ausgebildet sein.

Das untere Ende des Rückenbandes 8 seinerseits ist am oberen Ende der Bandage 6 - vorzugsweise lösbar - befestigt, insbesondere mittels (nicht dargestelltem) Klettverschluss, oder anderen geeigneten Verschlusssystemen, beispielsweise mittels Haken und Ösen. Im letzteren Fall hat es sich als vorteilhaft erwiesen, zwei oder drei horizontale Reihen von Haken oder Ösen vorzusehen, um eine Anpassung an die Körperabmessungen des Patienten zu ermöglichen.

Die Bandage 6 bietet vorzugsweise die Möglichkeit des Einschiebens von Verstärkungsstäben 4 und/oder des Anbringens von über den Leibesumfang verlaufenden Verstärkungsbändern (5).

## Patentansprüche

1. An einer Lendenbandage (6) befestigbarer Geradehalter, insbesondere für Patienten mit Osteoporose-Beschwerden, gekennzeichnet durch zwei über die Schultern eines Patienten verlaufende Schulterbänder (1,1'), deren obere, im Nackenbereich (7) des Patienten liegende Enden (2,2') mit dem oberen Ende eines in Längsrichtung über den Rücken verlaufenden Rückenbandes (8) - gegebenenfalls lösbar - verbunden sind, und die unter den Achseln des Patienten hindurch auf seinen Rücken führen, wobei die beiden unteren Enden (3,3') der Schulterbänder (1,1') an einer zumindest den Lendenbereich des Patienten umgreifenden Bandage (6), - gegebenenfalls lösbar - befestigbar sind.

2. Geradehalter nach Anspruch 1, dadurch gekennzeichnet, dass das Rückenband (8) mit der Bandage (6), vorzugsweise lösbar, verbunden ist.

3. Geradehalter nach Anspruch 1 oder 2, dadurch gekennzeichnet, dass jeweils die beiden oberen und/oder die beiden unteren Enden (2,2'; bzw. 3,3') der Schulterbänder (1,1') miteinander verbunden sind.

4. Geradehalter nach einem der Ansprüche 1 bis 3, dadurch gekennzeichnet, dass die dem Körper des Patienten zugewandte Seite der Schulterbänder (1,1') und/oder des Rückenbandes (8) und/oder der Bandage (6) mit einem Textilmaterial beschichtet oder aus einem solchen gebildet ist, das zu mindestens 50% aus wenigstens einer der folgenden Fasern besteht: Polyvinylchlorid-, Polyester- und Acryl-Fasern, Polypropylen-, Polyamid- oder Wolle-Fasern, mit einem Anteil von weniger als 10% Baumwoll-, Zellwoll- und/oder Seiden-Fasern, insbesondere aus einem Material aus wenigstens 50% Polyvinylchlorid-Fasern, vorzugsweise aus mehr als 75% Polyvinylchlorid- und maximal 25% Acryl- und/oder Polyester-Fasern.

5. Geradehalter nach einem der vorhergehenden Ansprüche, dadurch gekennzeichnet, dass die Verbindungen bzw. Befestigungen mittels eines an sich bekannten Klettverschlusses, mittels Druckknöpfen oder mittels Haken und Ösen, bewerkstelligt sind.

6. Geradehalter nach einem der vorhergehenden Ansprüche, dadurch gekennzeichnet, dass die Schulterbänder (1,1') und/oder das Rückenband (8) in ihrer Längsrichtung durch Steppnähte und/oder Einlagen verstärkt bzw. im wesentlichen undehnbar ausgebildet sind.

7. Zweiteilige Orthese bestehend aus einem Geradehalter nach einem der vorhergehenden Ansprüche und einer wenigstens den Lendenbereich eines Patienten umgreifenden Bandage (6), dadurch gekennzeichnet, dass der Geradehalter über ein in Längsrichtung über den Rücken verlaufendes Rückenband (8) und über die beiden unteren Enden (3,3') der Schulterbänder (1,1') mit der Bandage (6), gegebenenfalls lösbar, verbunden ist.

8. Orthese nach Anspruch 7, dadurch gekennzeichnet, dass die Bandage (6) als Rumpf-Orthese mit Verstärkungsstäben (4) und/oder Verstärkungsbändern (5) ausgebildet ist.

9. Geradehalter nach einem der Ansprüche 1 bis 6, zur Verwendung als therapeutisches Hilfsmittel, vorzugsweise zur aktiven, flexiblen Stützung der Rückenwirbelsäule, insbesondere von Osteoporosepatienten.

10. Orthese nach Anspruch 7 oder 8, zur Verwendung als therapeutisches Hilfsmittel, insbesondere zur aktiven, flexiblen Stützung der Rückenwirbelsäule, insbesondere von Osteoporosepatienten.
